(19) Europäisches Patentamt
European Patent Office
Office européen des brevets

(11) **EP 4 814 172 A1**

(12) **EUROPEAN PATENT APPLICATION**
published in accordance with Art. 153(4) EPC

(43) Date of publication:
**30.09.2026 Bulletin 2026/40**

(21) Application number: **25761230.9**

(22) Date of filing: **20.02.2025**

(51) International Patent Classification (IPC):
***D01F 6/00*** (2006.01) ***A61M 1/36*** (2006.01) ***B01D 15/00*** (2006.01) ***B01J 20/26*** (2006.01) ***B01J 20/28*** (2006.01) ***D01F 6/16*** (2006.01) ***D01F 6/52*** (2006.01)

(52) Cooperative Patent Classification (CPC):
**A61M 1/36; B01D 15/00; B01J 20/26; B01J 20/28; D01F 6/00; D01F 6/16; D01F 6/52**

(86) International application number:
**PCT/JP2025/005803**

(87) International publication number:
**WO 2025/182756 (04.09.2025 Gazette 2025/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.02.2024 JP 2024029316**

(71) Applicant: **Toray Industries, Inc.**
**Tokyo 103-8666 (JP)**

(72) Inventors:
- **MAYUMI, Tomohiro**
  **Otsu-shi, Shiga 520-8558 (JP)**
- **SHIRATORI, Yota**
  **Otsu-shi, Shiga 520-8558 (JP)**
- **FUJIEDA, Hiroaki**
  **Otsu-shi, Shiga 520-8558 (JP)**
- **BABA, Takeshi**
  **Kamakura-shi, Kanagawa 248-8555 (JP)**
- **FUJITA, Masaki**
  **Otsu-shi, Shiga 520-8558 (JP)**

(74) Representative: **Prüfer & Partner mbB**
**Patentanwälte · Rechtsanwälte**
**Sohnckestraße 12**
**81479 München (DE)**

# (54) POROUS FIBER, FIBER BUNDLE, ADSORPTION COLUMN, AND METHOD FOR PRODUCING PHARMACEUTICAL USING SAME

(57) The purpose of the present invention is to provide a porous fiber, a fiber bundle, and an adsorption column incorporating the fiber bundle with which it is possible to efficiently adsorb primarily high molecular weight substances to be removed by adsorption which are larger than albumin. The present invention provides a porous fiber containing an organic polymer. The average pore diameter in a cross section of the fiber is 100-1,000 nm. The average pore diameter in the fiber outer surface is 100-10,000 nm. When the average pore diameter in an intermediate region of the fiber cross-section is denoted as Rm and the average pore diameter near the center is denoted as Ri, Rm/Ri is 0.85-1.15. The porosity of the fiber outer surface is 5-50%.

【Figure 1】

102
100
101
S5500 3.0kV x400 SE
100um

## Description

### Technical Field

The present invention relates to a porous fiber, a fiber bundle, an adsorption column, and a method of producing a pharmaceutical using the same.

### Background Art

As an adsorbent housed in an adsorption column to adsorb and remove a substance to be removed contained in a liquid to be treated, there are many adsorbents the inside of which is made porous for the purpose of increasing the surface area contributive to adsorption. As the form of the adsorbent, a bead shape or a fiber shape is general. As a fiber-shaped adsorbent, there are hollow fiber, solid fiber, or knitted fabric of solid fiber. Among these, hollow fiber and solid fiber are excellent in that the flow path of a liquid to be treated can be sufficiently secured in a case where the hollow fiber and the solid fiber are housed in an adsorption column.

For example, Patent Literature 1 discloses a porous hollow fiber type separation membrane in which the pore diameter is controlled for the purpose of separating a high molecular weight protein such as an immune complex from a medium molecular weight protein having a molecular weight equal to or less than that of albumin, a low molecular weight protein, and water.

Patent Literatures 2 and 3 disclose a porous solid fiber that adsorbs a protein having a lower molecular weight than albumin.

Patent Literature 4 lists a crystalline polymer represented by polyethylene and polypropylene and an amorphous polymer represented by polysulfone, polystyrene, and polymethyl methacrylate, and discloses a porous solid fiber having uniform pores near the outer surface of the fiber and at or near the center of the fiber.

Patent Literatures 5 and 6 disclose a porous hollow fiber type separation membrane made of polysulfone in which the pore diameter and porosity of the inner (outer) surface of the hollow fiber are controlled for the purpose of obtaining a sufficient mechanical strength and a filtration flow rate.

### Citation List

#### Patent Literature

Patent Literature 1: JP S58-76104 A
Patent Literature 2: JP 2014-207989 A
Patent Literature 3: WO 2017/188119
Patent Literature 4: JP H06-296860 A
Patent Literature 5: JP H07-258915 A
Patent Literature 6: JP 2004-098027 A

### Summary of Invention

#### Technical Problem

However, the hollow fiber type separation membrane described in Patent Literature 1 is different in that the substance to be removed contained in the liquid to be treated is removed by separation using the hollow shape of the hollow fiber type separation membrane.

Additionally, in order to adsorb and remove a high molecular weight protein with high efficiency, it is necessary to design the pore diameter inside the fiber to be large. However, generally, as the pore diameter is larger, the fiber density is lowered and the mechanical strength is lowered, whereby the spinnability tends to deteriorate, but Patent Literature 1 does not have a description for solving such a problem of the deterioration of spinnability.

The porous solid fiber described in Patent Literature 2 and Patent Literature 3 does not have a description of adsorbing and removing a high molecular weight protein larger than albumin, which is a medium molecular weight protein, with high efficiency. Thus, the literature has no description of solving the deterioration of spinnability, which is a problem due to a large pore diameter.

As the porous solid fiber described in Patent Literature 4, a crystalline polymer and an amorphous polymer are mentioned as examples, but the literature has no description for improving the deterioration of spinnability which is a

concern particularly in the amorphous polymer the fiber of which has a weak mechanical strength. Additionally, the literature has no description of controlling the porous structure of the fiber surface to improve the deterioration of spinnability.

The hollow fiber described in Patent Literature 5 and Patent Literature 6 is different in that the substance to be removed contained in the liquid to be treated is removed by separation using the hollow shape of the hollow fiber, similarly to Patent Literature 1. Additionally, although the literature has a description regarding the pore diameter and porosity of the inner (outer) surface of the fiber, regarding the pore diameter in the cross section of the fiber, the porous structure is such that the pore diameter continuously changes from the outer surface toward the inner surface, and from the viewpoint of causing the pores to adsorb the substance to be removed, the structure cannot be said to be optimal.

In view of this, an object of the present invention is to provide a porous fiber, a fiber bundle, and an adsorption column having the fiber bundle, which are capable of highly efficiently adsorbing a substance to be removed by adsorption, such as a high molecular weight protein mainly larger than albumin.

Additionally, another object is to provide a porous fiber, a fiber bundle, and an adsorption column having the fiber bundle, which have high mechanical strength and excellent spinnability despite the large pore diameter of the fiber.

## Solution to Problem

The configuration of the present invention for solving the above problems is any one of the following (1) to (13).

(1) A porous fiber including an organic polymer, wherein the fiber has an average pore diameter in a cross section of 100 to 1000 nm, wherein the fiber has an average pore diameter in an outer surface of 100 to 10,000 nm, wherein Rm/Ri is 0.85 to 1.15, wherein Rm is average pore diameter in an intermediate region of the cross section of the fiber, and Ri is average pore diameter at or near the center of the cross section of the fiber, and wherein the outer surface of the fiber has a porosity of 5 to 50%.
(2) The porous fiber according to (1), wherein the porosity of the outer surface of the fiber is 5 to 40%.
(3) The porous fiber according to (1) or (2), wherein aspect ratio of major axis portion to minor axis portion of a pore in the outer surface of the fiber is 1.5 to 10.
(4) The porous fiber according to any one of (1) to (3), wherein the fiber has a dispersion component of a surface free energy of 5 to 100 mJ/$m^2$.
(5) The porous fiber according to any one of (1) to (4), wherein the organic polymer is an amorphous polymer.
(6) The porous fiber according to (5), wherein the amorphous polymer is an acrylic polymer.
(7) The porous fiber according to (5), wherein the amorphous polymer is polymethyl methacrylate.
(8) The porous fiber according to any one of (1) to (7), wherein the cross section of the fiber has a region A and a region B separated by a region boundary, wherein the region A is a uniform structure having an average pore diameter Ap of 100 nm to 1000 nm, and wherein an average pore diameter Bp of the region B is smaller than the average pore diameter Ap, or the region B is non-porous.
(9) The porous fiber according to any one of (1) to (8), wherein the porous fiber is a solid fiber.
(10) A fiber bundle including the porous fiber according to any one of (1) to (9).
(11) An adsorption column including the fiber bundle according to (10).
(12) The adsorption column according to (11), for use in the blood purification.
(13) A method of producing a pharmaceutical, including a purification step using the adsorption column according to (10) or (11).

## Advantageous Effects of Invention

The porous fiber of the present invention has excellent spinnability despite a large pore diameter. A fiber bundle including the porous fiber and an adsorption column having the fiber bundle make it possible to highly efficiently adsorb a high molecular weight protein or the like contained in a liquid to be treated.

## Brief Description of Drawings

[FIG. 1] FIG. 1 is a diagram showing an example of a scanning electron microscope image in which a virtual region 1 and a virtual region 2 are distinguished by a difference in lightness.
[FIG. 2] FIG. 2 is an enlarged view of a virtual region boundary portion of FIG. 1.
[FIG. 3] FIG. 3 is a schematic diagram showing a pore diameter line profile on a perpendicular line to a tangent line at an arbitrary point of the virtual region boundary of FIG. 2.

[FIG. 4] FIG. 4 shows an example of a change in a cross-sectional pore diameter at a region boundary, and includes: a schematic diagram in a case where the cross-sectional pore diameter changes discontinuously (FIG. 4-A); a schematic diagram in a case where the cross-sectional pore diameter changes continuously (FIG. 4-B); and a schematic diagram in a case where there is a portion having a locally large or small cross-sectional pore diameter (FIG. 4-C).

[FIG. 5] FIG. 5 is an example of an X-ray scattering spectrum in which a peak derived from a microcrystal overlaps with a peak derived from an amorphous portion to form a shoulder peak.

[FIG. 6] FIG. 6 is an example of an ultra-small angle X-ray scattering spectrum of a spinning dope in a case where X-ray scattering is measured.

Description of Embodiments

Details of the porous fiber, the fiber bundle, and the adsorption column of the present invention will be described below.

The porous fiber of the present invention is characterized by including an organic polymer, wherein the fiber has an average pore diameter in an outer surface of 100 to 10000 nm, wherein the fiber has an average pore diameter in a cross section of 100 to 1000 nm, and wherein Rm/Ri is 0.85 to 1.15, wherein Rm is average pore diameter in an intermediate region of the cross section of the fiber, and Ri is average pore diameter at or near the center of the cross section of the fiber, and wherein the outer surface of the fiber has a porosity of 5% to 50%.

The "cross section of the fiber" refers to a plane perpendicular to the longitudinal direction of a fiber in the porous fiber of the present invention.

The "outer surface of the fiber" is the surface on the outer peripheral side in the porous fiber of the present invention, that is, the outer peripheral portion in a cross section of the fiber.

Porous fiber

The fiber shape of the porous fiber of the present invention may be a hollow fiber having a hollow portion or a solid fiber not having a hollow portion, but the solid fiber is preferable because the solid fiber has a high fiber volume per unit volume and thus is excellent in adsorption performance, and has a high mechanical strength and is excellent in spinnability.

In the porous fiber of the present invention, "an average pore diameter in an outer surface of the fiber" and "an average pore diameter in a cross section of the fiber" may each be appropriately controlled according to the size of a substance to be removed by adsorption, but from the viewpoint of preventing the blockage of pores and the viewpoint of utilizing the inside of the porous fiber for highly efficient adsorption, two designs shown below are preferable.

That is, it is preferable to design each average pore diameter to be larger than the size of the substance to be removed by adsorption. Additionally, it is preferable to design the average pore diameter in the outer surface of the fiber to be larger than the average pore diameter in the cross section of the fiber. Note that these average pore diameters can be controlled by the type and concentration of a spinning dope, the temperature and humidity of a dry section, the temperature of a coagulation bath, and the like, which are described later during spinning.

Examples of the substance to be removed by adsorption by the porous fiber of the present invention include a substance to be removed by adsorption that has a weight average molecular weight of several hundreds of thousands to several millions and a size of approximately 15 nm to 100 nm, such as LDL cholesterol, an immune complex, endotoxin, and proteoglycan, which are described later.

From the viewpoint of highly efficiently adsorbing the substance to be removed by adsorption, the average pore diameter in the outer surface of the fiber is preferably 100 nm or more, more preferably 200 nm or more, and still more preferably 300 nm or more. On the other hand, from the viewpoint of maintaining the mechanical strength of the fiber, the average pore diameter in the outer surface of the fiber is preferably 10,000 nm or less, more preferably 5000 nm or less, and still more preferably 1000 nm or less. Here, in a case where a pore in the outer surface of the fiber is not circular, the average pore diameter in the outer surface of the fiber can be calculated by using an equivalent diameter obtained from a circle having the same area as the area surrounded by the outer periphery of the pore.

Cross section of fiber

Additionally, from the viewpoint of highly efficiently adsorbing the substance to be removed by adsorption, the average pore diameter in the cross section of the fiber is preferably 100 nm or more, more preferably 125 nm or more, and still more preferably 150 nm or more. On the other hand, from the viewpoint of maintaining the mechanical strength of the fiber, the average pore diameter in the cross section of the fiber is preferably 1000 nm or less, more preferably 750 nm or less, and still more preferably 500 nm or less. Here, in a case where a pore in the cross section of the fiber is not circular, the

average pore diameter in the cross section of the fiber can be calculated by using an equivalent diameter obtained from a circle having the same area as the area surrounded by the outer periphery of the pore.

Regarding "the average pore diameter in the cross section of the fiber" in the present invention, the inventors have found that it is important for highly efficiently adsorbing the substance to be removed by adsorption that the average pore diameter Rm of the intermediate region located between the outer surface and the center is approximately the same as the average pore diameter Ri at or near the center.

For a separation membrane employing the principle of filtration, a porous fiber in which the pore diameter in a cross section of the fiber continuously changes from the outer surface of the fiber toward the inside thereof is preferably used from the viewpoint of preventing clogging, as described in Patent Literature 6. However, in a case where a target substance is removed by adsorption, it is preferable that a pore diameter suitable for adsorbing the substance to be removed by adsorption is uniformly distributed from the intermediate region to the region at or near the center of the fiber because such a pore diameter enables the substance to be adsorbed with high efficiency. Specifically, the ratio of Rm/Ri is preferably 0.85 to 1.15, and more preferably 0.90 to 1.10.

On the other hand, for "the average pore diameter in the cross section of the fiber" in the present invention, it is preferable that an average pore diameter near the outer surface is made larger than the average pore diameter at or near the center. This makes it possible to provide a porous fiber that is capable of highly efficiently adsorbing the substance to be removed by adsorption, and has high mechanical strength and excellent spinnability.

Specifically, the value of Ro/Ri is preferably 1.3 or more, more preferably 1.4 or more, and still more preferably 1.5 or more, from the viewpoint of ensuring the spinnability, wherein Ro is the average pore diameter near the outer surface, and Ri is the average pore diameter at or near the center. On the other hand, from the viewpoint of highly efficient adsorption, the value of Ro/Ri is preferably 4.0 or less, more preferably 3.5 or less, and still more preferably 3.0 or less.

Additionally, from the viewpoint of highly efficiently adsorbing the substance to be removed by adsorption, Ro is preferably 150 nm or more, more preferably 180 nm or more, and still more preferably 200 nm or more. On the other hand, from the viewpoint of maintaining the mechanical strength of the fiber, Ro is preferably 1000 nm or less, more preferably 800 nm or less, and still more preferably 700 nm or less. Here, in a case where a pore in the cross section of the fiber is not circular, the average pore diameter in the cross section of the fiber can be calculated by using an equivalent diameter obtained from a circle having the same area as the area surrounded by the outer periphery of the pore.

Furthermore, from the viewpoint of highly efficiently adsorbing the substance to be removed by adsorption, Ri is preferably 100 nm or more, more preferably 120 nm or more, and still more preferably 140 nm or more. On the other hand, from the viewpoint of maintaining the mechanical strength of the fiber, Ri is preferably 1000 nm or less, more preferably 750 nm or less, and still more preferably 500 nm or less. Here, in a case where a pore in the cross section of the fiber is not circular, the average pore diameter in the cross section of the fiber can be calculated by using an equivalent diameter obtained from a circle having the same area as the area surrounded by the outer periphery of the pore.

In the porous fiber, a region "near the outer surface" is a region closer to the outer peripheral side of the porous fiber, and refers to a region corresponding to a 10% portion on the outer peripheral side in a case where an arbitrary straight line is drawn from the center point to the outer periphery in a cross section of the fiber. Here, the center point refers to the center of gravity of the cross section of the fiber. The average pore diameter near the outer surface is a pore diameter of a region where the substance to be removed by adsorption contained in the liquid to be treated invades into pores from the outer surface of the fiber, and thus, the average pore diameter is an important factor largely affecting the adsorption efficiency of the substance to be removed by adsorption.

However, near the outer surface of the porous fiber of the present invention, a region of a slight dense layer existing on the outermost surface is excluded. A "dense layer" refers to a region in which a pore having a pore diameter of 15 nm or more does not exist in the cross section of the fiber.

In the porous fiber, a region "at or near the center" is a region closer to the center side of the porous fiber, and refers to a region corresponding to a 10% portion on the center side in a case where an arbitrary straight line is drawn from the center point to the outer periphery in a cross section of the fiber. A portion at or near the center corresponds to the core of the porous fiber, the average pore diameter at or near the center is an important factor largely affecting the mechanical strength.

Additionally, an "intermediate region" is a region located intermediately between the center and outer surface of the porous fiber. Specifically, in a case where an arbitrary straight line is drawn from the center point to the outer periphery in the cross section of the fiber, and where the straight line is divided into 10 equal parts to divide the cross section of the fiber into 10 small regions, the intermediate region refers to the fifth small region from the region close to the center point.

The cross-sectional shape of the porous fiber is not particularly limited, and may be a circular shape or an irregular shape other than the circular shape. Also in the case of the irregular shape, a region "near the outer surface" is defined as a region corresponding to a 10% portion on the outer peripheral side in a case where an arbitrary straight line is drawn from the center point to the outer periphery of the cross section of the fiber, a region "at or near the center" is defined as a region corresponding to a 10% portion on the center side in a case where an arbitrary straight line is drawn from the center point to the outer periphery of the cross section of the fiber, and the "intermediate region" is defined as the fifth small region from the

region close to the center point in a case where an arbitrary straight line is drawn from the center point to the outer periphery in the cross section of the fiber, and where the straight line is divided into 10 equal parts to divide the cross section of the fiber into 10 small regions. The cross-sectional shape of the fiber can be controlled according to the shape of a spinneret that discharges the spinning dope.

In a case where the porous fiber is a hollow fiber, a region " near the outer surface", a region "at or near the center", and the "intermediate region" are defined excluding a hollow portion. That is, a region "near the outer surface" is a 10% portion on the outer peripheral side excluding the hollow portion in a case where an arbitrary straight line is drawn from the center point to the outer periphery of the cross section of the fiber, and a region "at or near the center" is a 10% portion on the center side excluding the hollow portion. Additionally, the "intermediate region" refers to the fifth small region from the region close to the center point in a case where an arbitrary straight line is drawn from the center point to the outer periphery in the cross section of the fiber, and where the straight line is divided into 10 equal parts excluding the hollow portion, to divide the cross section of the fiber into 10 small regions.

All of these average pore diameters can be calculated by performing image analysis on an image obtained by observing the outer surface of the fiber or the cross section of the fiber with a scanning electron microscope or the like, as described in Examples below.

Note that the porous fiber of the present invention may contain a third component such as fine particles inside thereof, as a pore-forming agent or an adsorbent. However, since there is a risk of lowering the mechanical strength of the porous fiber, deteriorating cleaning efficiency during spinning, or eluting the third component from the inside of the porous fiber to the outside, it is preferable that the porous fiber does not contain the third component.

It is also one preferable aspect that the porous fiber of the present invention is a porous fiber having: a region A which is a uniform structure of 100 nm to 1000 nm; and a region B separated by a region boundary, wherein the average pore diameter Bp of the region B is smaller than the average pore diameter Ap of the region A, or is non-porous. By making such a composite porous fiber having two regions with different pore sizes, it is possible to obtain a fiber having a higher mechanical strength than a porous fiber having only the region A.

Here, a method of determining the region A, the region B, and the region boundary will be described.

First, an approximate position of the region boundary which is a boundary between the region A and the region B is determined at a magnification at which the entire cross section of the fiber can be observed. This position is called a virtual region boundary 102, and regions separated by the virtual region boundary are called a virtual region 1 (100) and a virtual region 2 (101).

Next, the virtual region boundary is magnified and observed at a magnification at which the porous structure can be observed, and the precise positions of the respective boundaries of the boundary 1/the region boundary and the region boundary/the boundary 2 are determined. Then, cross-sectional pore diameters of a region 1 and a region 2 are obtained, and one having a larger cross-sectional pore diameter is determined as the region A, and the other having a smaller cross-sectional pore diameter is determined as the region B.

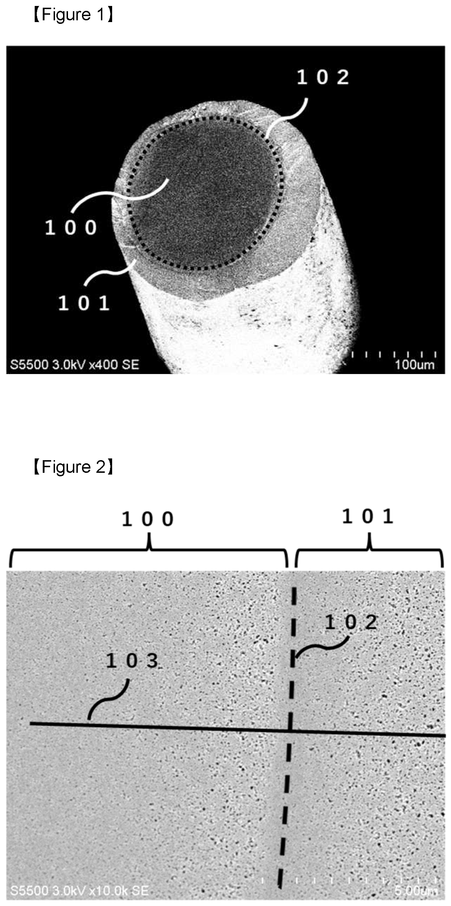

Hereinafter, specific description will be given by taking FIG. 1 to FIG. 3 as examples. The cross section of the porous fiber is observed with a scanning electron microscope (for example, S-5500 manufactured by Hitachi High-Technologies Corporation) at a magnification at which the entire cross section of the fiber can be observed. In the porous fiber of the present invention, the region A preferably has a uniform structure, and the inside of the region A can be observed with homogeneous lightness. Additionally, since the region B preferably has a smaller cross-sectional pore diameter than the region A, the virtual region 1 (100) and the virtual region 2 (101) can be distinguished by the virtual region boundary 102 by virtue of a difference in lightness, as shown in FIG. 1. In a case where materials constituting the region A and the region B are identical, where a difference in cross-sectional pore diameter is small (for example, approximately 10 nm), and where a difference in lightness between the regions is small, the contrast and the brightness are appropriately adjusted so that the difference in lightness between the regions can become large. For the distinguished regions, the virtual region boundary is determined visually or by image processing. In the case of the visual observation, a place where lightness changes visually is regarded as the virtual region boundary. In the case of image processing, binarization is performed such that, out of the two regions, the region having a higher lightness becomes white, and that the region having a lower lightness becomes black, and the boundary therebetween is regarded as the virtual region boundary. In this case, determination is performed ignoring the following: unevenness generated when a cross section of the porous fiber is produced; lightness and darkness derived from a scratch; and/or lightness and darkness derived from noise. In the case of image processing, binarization is performed after an appropriate image processing such as a smoothing processing or a noise removal is applied depending on the situation.

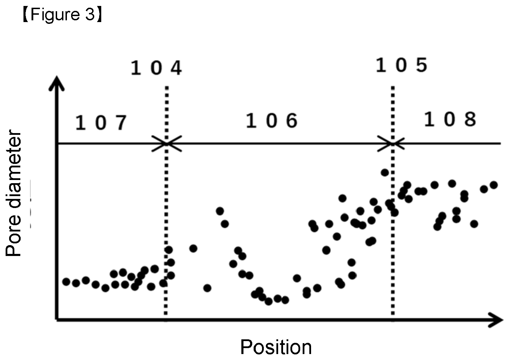

After determining the virtual region boundary, the magnification is increased so as to enable the porous structure to be observed, and a place where the porous structure changes is determined as the accurate boundary of the region. The place where the porous structure changes is determined by the following image processing method. The pore diameters of pores existing on a line profile of a perpendicular line 103 to a tangent line at an arbitrary point of the virtual region boundary 102 as shown in FIG. 2 are plotted as shown in FIG. 3. Here, by using image analysis software (for example, ImageJ), the diameter of a circle obtained by fitting a pore with a circle is set as a pore diameter, and the center of the circle is set as the

position of the pore. As shown in FIG. 3, the pore diameter takes a constant value within a range of variation in each region, but changes at a certain position. On the virtual region 1 side, a position where a change in this pore diameter profile occurs is set as the accurate position of a boundary, that is, a boundary 104 of the region 1, and on the virtual region 2 side, a position where a similar change occurs is set as a boundary 105 of the region 2, and a region between the boundary 104 of the region 1 and the boundary 105 of the region 2 is set as a region boundary 106.

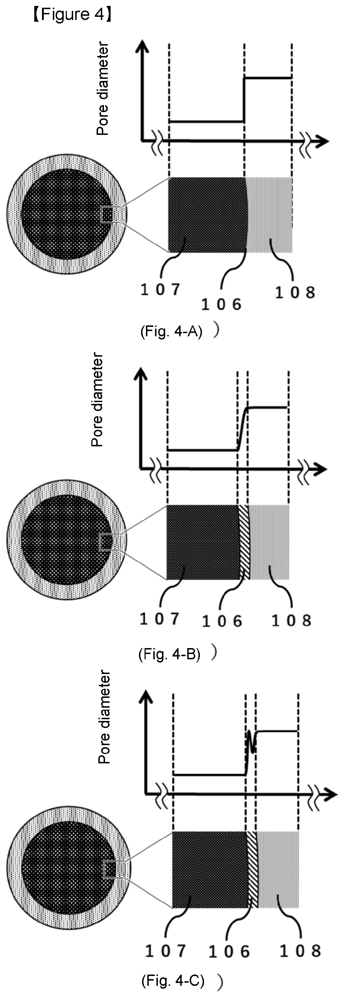

The "region boundary" means a region separating the region 1 and the region 2. In the region boundary, the cross-sectional pore diameter may change discontinuously (FIG. 4-A), or may change continuously (FIG. 4-B). Additionally, there may be a portion having a locally large or small cross-sectional pore diameter (FIG. 4-C).

For each region determined by the above method, one having a larger cross-sectional pore diameter obtained by a method described in "Average Pore Diameter in Cross Section of Fiber" described later is set as a region A, and the other having a smaller cross-sectional pore diameter is set as a region B. Note that the region A and/or the region B may be present at a plurality of locations in the cross section of the fiber, and in this case, similar measurement is performed for each location.

An adsorption principle of the substance to be removed by adsorption by the porous fiber of the present invention may be any of a hydrophobic interaction, an electrostatic interaction, a hydrogen bond, and the like, and a plurality of principles may be combined. The hydrophobic interaction has a sufficient force for adsorbing a protein and the like, causes a smaller effect on blood cells and the like, and thus, is particularly preferable. These adsorption principles are basically determined according to the below-described material of the porous fiber.

Note that a plurality of ligands that specifically interact with the substance to be removed by adsorption may be immobilized on the outer surface of the fiber and inside pores of the porous fiber. On the other hand, it is difficult to simultaneously immobilize a plurality of ligands on a porous fiber. Even if successfully immobilized, the ligands can compete with each other, resulting in the risk of decreasing the ligand immobilization amount and decreasing the adsorption performance. Thus, it is preferable that the number of the types of the ligands to be immobilized on the porous fiber is one or two.

The material of a region near the outer surface of the porous fiber of the present invention and the material of a region at or near the center may be the same or different. However, the respective materials of the regions are preferably the same from the viewpoint of preventing delamination, deformation of the porous fiber, and the like resulting from differences in physical or chemical properties due to material variations.

In the porous fiber of the present invention, the outer surface of the fiber and the pore surface inside the fiber desirably have a moderate hydrophobicity from the viewpoint of achieving both blood compatibility and adsorption performance. The hydrophobicity can be quantified with a dispersion component of surface free energy. Specifically, a dispersion component of surface free energy, as measured by inverse gas chromatography, is preferably 5 to 100 $mJ/m^2$, more preferably 10 to 80 $mJ/m^2$, and still more preferably 20 to 70 $mJ/m^2$. As a material of the porous fiber of the present invention, an organic polymer is preferably used from the viewpoint of molding processability, cost, and the like. Examples of the organic polymer include polymethyl methacrylate (hereinafter referred to as "PMMA"), polyacrylonitrile (hereinafter referred to as "PAN"), polyacrylamide, polysulfone, polyethersulfone, polyarylethersulfone, polypropylene, polystyrene, polycarbonate, polylactic acid, polyethylene terephthalate, cellulose, cellulose triacetate, ethylene-vinyl alcohol copolymer, polycaprolactam, polymethylpentene, polyvinylidene fluoride, polyester polymer alloy, or a derivative thereof. Among these, PMMA, PAN, polysulfone, polyethersulfone, a polyester polymer alloy, polystyrene, or cellulose triacetate, having a moderate hydrophobic interaction, is preferably used, and PMMA is particularly preferably used.

Amorphous polymer

The porous fiber of the present invention preferably contains an amorphous polymer, and more preferably contains the amorphous polymer as a main component. Here, an "amorphous polymer" is a polymer that solidifies with the molecules randomly entangled, when cooled from a temperature at which the polymer is in a molten state. Here, "containing the amorphous polymer as a main component" means that the amorphous polymer accounts for 50% by weight or more of the whole components constituting the porous fiber. The amorphous polymer is particularly excellent from the viewpoint of molding processability and cost, has a uniform structure even from a microscopic viewpoint, and can be expected to undergo a uniform adsorption reaction on a surface.

Additionally, regarding the physical or chemical properties of a porous fiber, an amorphous polymer is easy to handle as a material. Among amorphous polymers, PMMA is particularly preferable because PMMA has moderate hydrophobicity, is excellent in mechanical strength, and can withstand radiation sterilization and the like in use for medical purposes.

In a case where the porous fiber of the present invention contains an amorphous polymer, it is preferable that a microcrystal derived from the amorphous polymer exists in the porous fiber in order to obtain a porous fiber having high mechanical strength and excellent spinnability even if the average pore diameter in the outer surface of the fiber or in the cross section of the fiber is controlled to be large. Generally, an amorphous polymer is unlikely to form a crystal, but can be

partly crystallized by a physical method such as stretching or pressurization, a method employing a specific solvent, a method of controlling the stereoregularity of a polymer, or the like. For example, in some of the cases where a polymer has different optical isomers, and these form a complex (hereinafter referred to as a "stereocomplex"), a microcrystal is formed.

The microcrystal derived from the amorphous polymer in the present invention can be confirmed by analyzing the crystal structure of the porous fiber, as described in Examples below.

An X-ray scattering measurement is used as a technique for analyzing the crystal structure. Compared to a laboratory-level X-ray diffraction apparatus (for example, SmartLab manufactured by Rigaku Corporation), a wide-angle X-ray scattering (hereinafter referred to as "WAXS") employing a high-brilliance synchrotron radiation facility (for example, SPring-8) is particularly preferable because of high sensitivity.

As a means of judging whether or not a microcrystal exists in a porous fiber, in the case of a known microcrystal, it is possible to judge that a microcrystal exists if a peak is seen at a peak position derived from a microcrystal in light of literature in which X-ray scattering data of the microcrystal are described. In the case of an unknown microcrystal, it is possible to judge whether a peak is derived from a microcrystal, by performing an X-ray structural analysis based on a diffraction pattern obtained by WAXS.

In order to have excellent spinnability despite having a large pore diameter, and adsorbs, with high efficiency, a high molecular weight protein or the like contained in a liquid to be treated, in the porous fiber of the present invention, the lattice spacing (d) of the microcrystal is preferably within an optimal range. The scattering vector q and the lattice spacing d are in the relation $q = 2\pi/d$.

In order to achieve high mechanical strength, it is preferable that a peak of a scattering vector (q) derived from a microcrystal of an amorphous polymer, as obtained by an X-ray structural analysis of WAXS, exists at less than 1.2 $Å^{-1}$. This means that the lattice spacing d of the microcrystal of the amorphous polymer is relatively large compared to a crystal having a folded structure generally observed in a crystalline polymer. That is, the amorphous polymer has a more complicatedly entangled crystal structure, and thus, is unlikely to form a crystal. However, if a microcrystal is once formed, for example, by the below-described method employing a hydrogen bond, a stable crystal structure in which amorphous polymers entangled therebetween are unlikely to be disentangled is formed, so that a porous fiber having a high mechanical strength can be obtained.

On the other hand, if the lattice spacing is excessively large, it becomes difficult to form a microcrystal. Thus, in the porous fiber of the present invention, a peak of a scattering vector (q) derived from a microcrystal of an amorphous polymer, as obtained by an X-ray structural analysis of WAXS, preferably exists at 0.2 $Å^{-1}$ or more.

In particular, since a microcrystal is easily formed, and a high mechanical strength is obtained, it is preferable that a peak derived from a microcrystal of an amorphous polymer, as obtained by an X-ray structural analysis of WAXS, is observed at any of 0.29, 0.84, and 1.14 $Å^{-1}$.

The amorphous polymer contained in the porous fiber of the present invention is preferably an acrylic polymer. In particular, an acrylic polymer such as PMMA, PAN, or polyacrylamide is more preferable because such an acrylic polymer is excellent in blood compatibility, and in addition, the function of the polymer is easy to exhibit by modifying a terminal group thereof. Among these, PMMA is still more preferable from the viewpoint that PMMA has different optical isomers-syndiotactic polymers and isotactic polymers-despite being an amorphous polymer, and that the isomers form a stereocomplex.

Porosity

The porosity of the outer surface of the porous fiber of the present invention is preferably 5% or more, more preferably 10% or more, and still more preferably 15% or more, from the viewpoint of highly efficiently adsorbing the substance to be removed by adsorption. On the other hand, the porosity of the outer surface of the porous fiber is preferably 50% or less, more preferably 40% or less, and still more preferably 35% or less from the viewpoint of being able to expect to have the effect of preventing a decrease in the mechanical strength of the fiber, preventing a non-selective adsorption, or preventing fine particles and the like generated inside the pores from flowing out to the outside of the fiber.

Additionally, for the pores in the outer surface of the fiber in the present invention, the aspect ratio of the major axis (the longest radius of the pores) to the minor axis (the shortest radius of the pores) is important. In a case wherein Rl is the major axis, and Rs is the minor axis, the value of Rl/Rs is preferably 1.5 or more, more preferably 2.5 or more, and still more preferably 4.0 or more, in order to highly efficiently adsorb and remove the substance to be removed by adsorption. On the other hand, from the viewpoint of maintaining the mechanical strength of the fiber, the value of Rl/Rs is preferably 10 or less, more preferably 8 or less, and still more preferably 7 or less.

Both of these porosity and aspect ratio of the outer surface of the fiber can be calculated by performing an image analysis on an image obtained by observing the outer surface of the fiber with a scanning electron microscope or the like, as described in Examples below.

Method of producing porous fiber

The method of producing the porous fiber according to the present invention may be either melt spinning or solution spinning. A technique in which a composite fiber obtained by melt spinning is made porous by a post-treatment such as stretching or extraction is also conceivable. However, in a case where the fiber is made porous by stretching, the value of Rl/Rs in the outer surface of the fiber becomes extremely large, likely making it more difficult to maintain mechanical strength. Additionally, in a case where the fiber is made porous by extraction, the process tends to be complicated.

On the other hand, in solution spinning, a fiber having a target porous structure can be obtained by removing a solvent from a state in which a fiber material is uniformly dissolved in the solvent. Additionally, making the fiber porous by a post-treatment such as melt spinning is unnecessary, productivity is excellent, and concerns about peeling or breaking are extremely small. For these reasons, the method of producing the porous fiber according to the present invention is preferably solution spinning.

Additionally, in order to control the surface structure and pore size of the porous fiber, a method utilizing phase separation between an organic polymer and a solvent in a spinning dope in solution spinning is preferably used. Examples of the technique utilizing phase separation include a thermally induced phase separation method and a non-solvent induced phase separation method, and which method is suitable varies depending on the material of an organic polymer and a desirable pore diameter range.

However, in the present invention, it is preferable to use the thermally induced phase separation method that can be controlled among a dope producing step, a discharging step, and a cooling step, which are described later. It is more preferable to combine the thermally induced phase separation method and the non-solvent induced phase separation method, which methods can be controlled including the below-described coagulation step, in addition to the dope producing step, the discharging step, and the cooling step.

Hereinafter, a method of producing a porous fiber obtained by solution spinning will be described, but the method is not limited thereto.

The porous fiber of the present invention can be obtained by a producing method including a "dope producing step of obtaining a spinning dope by dissolving an organic polymer in a solvent", followed by a "discharging step of heating the spinning dope to a gel point or higher and discharging the spinning dope through a spinneret", followed by a "cooling step of cooling the spinning dope to the gel point or lower in a dry section after being discharged through the spinneret", and a "coagulation step of forming a porous fiber by a coagulation bath containing a compound that is incompatible with the organic polymer in the spinning dope", wherein the spinning dope has a scattering at a scattering vector (q) = 1 $nm^{-1}$ or less in an ultra-small angle X-ray scattering measurement under a temperature condition higher than the gel point.

The "dope producing step of obtaining a spinning dope by dissolving an organic polymer in a solvent" refers to a step of obtaining a spinning dope by dissolving a solid organic polymer in a solvent. A method of dissolving a solid organic polymer is not particularly limited, but a method of putting an organic polymer and a solvent into a container, and performing heating and stirring until a uniform solution is formed is suitably used. The form of the solid is not particularly limited, but is preferably powdery or flaky to enhance solubility. The spinning dope in the present invention has a gel point from the viewpoint of improving the spinnability and controlling the pore size.

The "discharging step of heating the spinning dope to a gel point or higher and discharging the spinning dope through a spinneret" refers to a step of heating the spinning dope obtained in the dope producing step to the gel point of the spinning dope or higher and discharging the spinning dope through the spinneret. By heating the spinning dope to the gel point or higher, the fluidity of the spinning dope increases, and a good discharge property through the spinneret is obtained. Here, the gel point means a temperature at which a liquid-state spinning dope having fluidity at a high temperature transitions to a gel state having no fluidity in a case where the spinning dope is cooled. Examples of a method of determining a gel point include visual observation or viscosity measurement, but viscosity measurement is used in the present invention. In the case of viscosity measurement, a temperature at which the viscosity rapidly increases can be determined as a gel point. Examples of the method of viscosity measurement include a rheometer and a falling ball method.

The "cooling step of cooling the spinning dope to the gel point or lower in a dry section after being discharged through the spinneret" refers to a step of cooling the spinning dope discharged through the spinneret to the gel point or lower. Thereby, the mechanical strength of a porous fiber obtained through the below-described coagulation step increases, and the spinnability can be improved. Additionally, the pore size can be controlled by a thermally induced phase separation method.

The "coagulation step of forming a porous fiber by a coagulation bath containing a compound that is incompatible with the organic polymer in the spinning dope" refers to a step of introducing, into a coagulation bath, the spinning dope (a precursor of a porous fiber, containing a solvent) having undergone the above discharging step and cooling step, causing substance exchange between the solvent used in the dope producing step and the compound incompatible with the organic polymer and contained in the coagulation bath, and precipitating an organic polymer to form a porous fiber.

In the present invention, the spinning dope can influence a porous structure in a cross section of the fiber and in the outer surface of the fiber in the obtained porous fiber, by controlling the nanodomain structure in the spinning dope and derived from phase separation or gelation.

In the present invention, it is preferable that the spinning dope has a scattering at a scattering vector (q) = 1 $nm^{-1}$ or less in an ultra-small angle X-ray scattering measurement under a temperature condition higher than the gel point of the spinning dope. Having a scattering at a scattering vector (q) = 1 $nm^{-1}$ or less in an ultra-small angle X-ray scattering measurement means that a nanodomain structure of several nanometers or more is formed in the spinning dope. That is, in the spinning dope, a nanodomain structure is already formed in the discharging step through a spinneret heated to a gel point or higher, and the nanodomain structure grows and is immobilized through the cooling step and the coagulation step, whereby the porous structure of the porous fiber is determined.

Here, the "growth of a nanodomain structure" means that a concentration difference between a polymer-dilute phase and a polymer-rich phase that form a nanodomain structure becomes large, or the size of the nanodomain structure becomes large. The immobilization of a nanodomain structure means that in the coagulation step, the growth of the nanodomain structure stops by an exchange between the solvent of the spinning dope and the solvent of the coagulation bath, and the structure settles in a constant state. On the other hand, in the case of not having a nanodomain structure under a temperature condition higher than the gel point, formation of a nanodomain structure starts after being cooled to the gel point or lower in the cooling step. In the spinning dope, the organic polymer is in a state in which mobility is relatively lowered at a gel point or lower, the growth of a nanodomain structure is suppressed, and the fiber becomes a fiber having a smaller pore diameter than the porous fiber of the present invention.

By using this spinning dope and performing the below-described "discharging step of heating the spinning dope to a gel point or higher and discharging the spinning dope through a spinneret" and "cooling step of cooling the spinning dope to the gel point or lower in a dry section after being discharged through the spinneret", a nanodomain structure is grown and immobilized, and pores for highly efficiently adsorbing a high molecular weight protein are formed.

A spinning dope in the present invention may be composed of only an organic polymer and a good solvent, but preferably contains a poor solvent for the organic polymer, in addition to the organic polymer and the good solvent. Here, the good solvent refers to a solvent capable of dissolving an organic polymer, and the poor solvent refers to a solvent that does not dissolve an organic polymer even when heated to a high temperature. This is to control phase separation, that is, to control formation of the nanodomain structure, by adjusting the affinity of the organic polymer to the solvent through addition of a poor solvent, rather than by forming a uniform state in which the organic polymer and the good solvent therefore are completely compatible.

As a material of an organic polymer used in the dope producing step, an organic polymer exemplified as a material of the porous fiber is preferably used.

Examples of the solvent used in the dope producing step include a good solvent for the organic polymer. As a good solvent, only one type can be used, or a plurality of types can be mixed and used, but from the viewpoint of cost and resource recovery, it is preferable to use only one type.

In a case where the organic polymer is PMMA, specific examples of a good solvent include: aromatic compounds such as benzene, toluene, and xylene; ketone-based compounds such as acetone; alcohol compounds such as methanol, ethanol, iso-propanol, and benzyl alcohol; ester compounds such as ethyl acetate and butyl acetate; halogen-containing compounds such as chloroform, dichloromethane, and chlorobenzene; and tetrahydrofuran, dimethylformamide, dimethyl sulfoxide, dimethylacetamide, N-methylpyrrolidone, acetonitrile, and the like. Among these, dimethyl sulfoxide (hereinafter referred to as "DMSO") is preferably used because of having overall high solubility and a high boiling point.

The kind of the poor solvent is not particularly limited. In a case where the organic polymer is PMMA, preferable examples of the poor solvent include: water; alcohol compounds such as methanol, ethanol, n-propanol, iso-propanol, 1-butanol, ethylene glycol, propylene glycol, diethylene glycol, 1,3-propanediol, 1,4-butanediol, glycerin, and phenols; ether compounds such as diethyl ether, 1,4-dioxane, and diglyme; hydrocarbon compounds such as hexane and heptane; amino group-containing compounds such as ethanolamine and formamide; acetic acid, formic acid, mercaptoethanol, carbon disulfide, and the like.

Furthermore, for a relationship between the organic polymer and the poor solvent, it is important that there is an interaction such as a hydrophobic interaction, an electrostatic interaction, or a hydrogen bond for the purpose of controlling a nanodomain structure, promoting formation of a microcrystal of an organic polymer, and improving spinnability. A plurality of these principles may be combined, but a hydrogen bond, which has a moderate strength of interaction, is more preferable.

Specifically, it is preferable that the organic polymer is a polymer containing a hydrogen bond-donating functional group, and the poor solvent is a hydrogen bond-accepting compound, or that the organic polymer is a polymer containing a hydrogen bond-accepting functional group, and the poor solvent is a hydrogen bond-donating compound. Forming a hydrogen bond between the organic polymer and the poor solvent allows controlling directions of a hydrogen bond-forming functional group and a hydrogen bond-donating or accepting functional group that are present in the organic polymer. Thus, the higher-order structure of the organic polymer can be controlled. It is considered that this promotes formation of a

microcrystal of the organic polymer, resulting in increasing the mechanical strength of the porous fiber and improving the spinnability. Additionally, forming an interaction with the poor solvent makes it possible to properly control the mobility of the organic polymer, and to control formation of the nanodomain structure.

From these points, the poor solvent is preferably a compound containing a hydrogen bond-donating or accepting functional group.

As a specific compound containing a hydrogen bond-donating functional group, water or a compound having a hydroxyl group or an amino group is preferable without particular limitation. For example, water, ethylene glycol, propylene glycol, 1,4-butanediol, glycerin, or formamide is more preferable.

As a specific compound containing a hydrogen bond-accepting functional group, a ketone compound or an ester compound is preferable, and acetone, ethyl acetate, or butyl acetate is preferable. The poor solvent may be used by combining a solvent of any of these compounds containing a hydrogen-bond-donating functional group and compounds containing a hydrogen-bond-accepting functional group.

The weight ratio of the poor solvent is also not particularly limited, but by setting an appropriate weight ratio, the formation of the above-mentioned nanodomain structure can be sufficiently promoted, and the spinnability can be improved. The interactions and mobility vary depending on the types of the organic polymer, good solvent, and poor solvent, and accordingly, the appropriate weight ratio of the poor solvent varies. For example, in a case where PMMA is used as the organic polymer, DMSO is used as the good solvent, and water, an alcohol compound, or an amino group-containing compound is used as the poor solvent, the weight ratio of the poor solvent to the entire solvent is preferably 1% by weight or more, more preferably 3% by weight or more, and still more preferably 5% by weight or more. On the other hand, the weight ratio of the poor solvent to the entire solvent is preferably 25% by weight or less, more preferably 15% by weight or less, and still more preferably 10% by weight or less.

The weight ratio of the organic polymer to the entire spinning dope used in the dope producing step is not particularly limited, but from the viewpoint of maintaining the mechanical strength of the porous fiber obtained by promoting physical and chemical crosslinking typified by entanglement and gelation between organic polymers, the weight ratio of the organic polymer to the entire spinning dope is preferably 5% by weight or more, more preferably 7.5% by weight or more, and still more preferably 10% by weight or more. On the other hand, from the viewpoint of improving the dischargeability of the spinning dope and increasing the number of pores in the obtained porous fiber, the weight ratio of the organic polymer to the entire spinning dope is preferably 20% by weight or less, more preferably 18% by weight or less, and still more preferably 15% by weight or less.

Additionally, from the viewpoint of maintaining the mechanical strength of the obtained porous fiber by promoting physical and chemical crosslinking typified by entanglement and gelation between organic polymers, the weight-average molecular weight of the organic polymer in the spinning dope used in the dope producing step is preferably 10,000 or more, more preferably 50,000 or more, and still more preferably 100,000 or more.

On the other hand, from the viewpoint that discharge through the spinneret becomes difficult in the discharging step owing to the increased viscosity of the spinning dope, the weight-average molecular weight of the organic polymer in the spinning dope used in the dope producing step is preferably 10,000,000 or less, more preferably 5,000,000 or less, and still more preferably 3,000,000 or less.

The temperature of the discharging step is not particularly limited, but from the viewpoint of stably maintaining the nanodomain structure obtained in the dope producing step and stably discharging the dope through the spinneret with the concentration of the spinning dope appropriately maintained, the temperature of the discharging step is preferably increased to or the gel point of the spinning dope or higher. For example, in a spinning dope containing PMMA and DMSO, the temperature of the discharging step is preferably 70°C or higher, more preferably 80°C or higher, and still more preferably 85°C or higher. On the other hand, to avoid decomposition of the constituent components of the spinning dope, the temperature of the discharging step is preferably 150°C or lower, more preferably 130°C or lower, and still more preferably 110°C or lower.

In the cooling step, to obtain a porous fiber having a large pore diameter and excellent mechanical strength, it is important that the spinning dope after being discharged through the spinneret is cooled to the gel point or lower. The cooling rate and cooling time are not particularly limited, but natural cooling alone takes time to cool the dope to the gel point or lower and requires slowing the spinning speed, it is preferable to actively cool the dope by blowing cold air or the like. In the cooling step, the spinning dope after being discharged through the spinneret is cooled from the outer surface of the fiber, and thus, such cooling is particularly important for controlling the pore diameter in the outer surface of the porous fiber, the porosity of the outer surface of the fiber, and the pore diameter near the outer surface in a cross section. In the case of blowing cold air, the amount of the cold air is arbitrarily determined within a range that is sufficient to cool the dope to the gel point or lower and that can suppress the risk of yarn breakage.

In the coagulation step, the spinning dope (a precursor of the porous fiber containing a solvent) that has undergone the cooling step undergoes solvent exchange with a compound that is incompatible with the organic polymer and contained in the coagulation bath, and the organic polymer precipitates, whereby a porous fiber is obtained. Examples of the compound incompatible with the organic polymer include, but are not particularly limited to, water or alcohol

compounds.

The temperature of the coagulation bath is not particularly limited, but to stably maintain the porous structure, the temperature of the coagulation bath is preferably 100°C or lower, more preferably 90°C or lower, and still more preferably 80°C or lower. On the other hand, to promote solvent exchange, the temperature of the coagulation bath is preferably 10°C or higher, more preferably 20°C or higher, and still more preferably 30°C or higher.

Additionally, the method of producing a porous fiber according to the present invention may include a washing step, a heat treatment step, a stretching step, a glycerin bath step, and the like, in addition to the above steps.

Regarding the fiber bundle, by forming a fiber bundle containing the porous fiber of the present invention, a fiber bundle having good handleability and exhibiting high adsorption performance can be obtained. In a case where a fiber bundle is formed, in order to prevent the porous fibers from repelling each other owing to static electricity or the like and losing cohesion, or to prevent adhesion between single yarns, the fiber bundle may be wrapped with a film, net, mesh, nonwoven fabric, or the like, or a processed yarn called a covering yarn may be spirally wound around one or a plurality of fibers.

In the adsorption column of the present invention, the fiber bundle of the present invention is housed in a casing having ports communicating with the interior at both ends. Examples of the shape of the casing include: angular cylindrical bodies such as a square cylindrical body and a hexagonal cylindrical body; or a circular cylindrical body, in which all these bodies have open ends at both ends.

Among them, a circular cylindrical body, particularly a cylindrical body having a perfectly circular cross section in the radial direction, is preferable as the casing. This is because the casing having no corners can suppress the retention of blood at the corners. Additionally, by making both ends of the casing open, the flow of the liquid to be treated is less likely to be disturbed, and enables pressure loss to be minimized.

As the material of the casing, plastic, metal, and the like can be used, and among them, plastic is preferably used as the material of the casing from the viewpoints of cost, moldability, weight, blood compatibility, and the like.

In a case where plastic is used as the material of the casing, a thermoplastic resin excellent in mechanical strength and thermal stability is suitably used. Examples of the thermoplastic resin include polycarbonate resins, cellulose resins, polyester resins, polyarylate resins, polyimide resins, cyclic polysulfone resins, polyethersulfone resins, polyolefin resins, polystyrene resins, polyvinyl alcohol resins, ABS resins, or mixtures thereof.

Among them, from the viewpoints of moldability and radiation resistance required for the casing, the material of the casing is preferably polypropylene, polystyrene, polycarbonate, ABS resin, or a derivative thereof. In particular, a resin excellent in transparency, such as polystyrene or polycarbonate, is advantageous for ensuring safety, for example, because the internal state can be confirmed during perfusion of the liquid to be treated.

Both ends of the casing are preferably physically sealed. Examples of the sealing method include a method of arranging a mesh or a method of providing a through-hole communicating between the inside and outside of the casing by penetrating a partition wall after fixing with a resin or the like.

Here, the "through-hole" refers to an opening penetrating the partition wall in the longitudinal direction of the porous fiber. That is, the through-hole is a hole that exists in the partition wall and penetrates therethrough, communicating the inside and outside of the casing separated by the partition wall.

Here, the "partition wall" refers to a portion that separates the inside and outside of the casing at an end of the casing, and examples thereof include a resin or the like that fixes the casing and the fibers. In a case where a resin is used as the partition wall, the partition wall can be obtained by injecting a resin liquid such as polyurethane from both ends of the casing, curing the resin liquid while flowing it to the ends of the casing by centrifugal force, and then cutting off unnecessary portions.

Among the sealing methods, the method of arranging a mesh is simpler in process than the method of forming a partition wall, makes it easy to uniformly distribute the flow of the liquid to be treated in the column, and thus, is preferable. Additionally, in order to further enhance the dispersibility of the liquid to be treated in the column, a part of the mesh may be a mesh having a larger pressure loss, or be provided with, for example, a plate-like member for controlling the flow, the member called a baffle plate or a rectifying plate.

Examples of the fiber form in a case where the fiber bundle is housed in the casing include: a form in which porous fibers are finely cut; and a form in which fibers are highly processed into a knitted fabric, a woven fabric, a nonwoven fabric, or the like. Among these, a fiber bundle in which straight-shaped fibers are bundled is preferable, and it is preferable to insert the fiber bundle parallel to the longitudinal direction of the casing.

A fiber bundle in which straight-shaped porous fibers are bundled easily secures a flow path for the liquid to be treated, and thus, it is easy to uniformly distribute the liquid to be treated in the casing. Additionally, the flow is less likely to become turbulent, which is also advantageous against an increase in pressure loss. Accordingly, even in a case where highly viscous blood is used as the liquid to be treated, risks such as coagulation within the casing can be minimized.

The adsorption column of the present invention is capable of highly efficiently adsorbing a substance to be removed by adsorption that has a high molecular weight, and is mainly larger than albumin. Examples of applications thereof include: filters for various fluids regardless of whether the fluids are in a gaseous or liquid phase; adsorbents; heat

insulators; sound absorbing materials; impact buffering materials; base materials for cell culture; and carriers for regenerative medicine. For example, in medical applications, the adsorption column is suitably used for the removal of pathogenic proteins and the like from blood, plasma, and body fluids and for the removal of impurities from biopharmaceutical raw material solutions in the purification of pharmaceuticals, particularly in the purification process of biopharmaceuticals.

Hereinafter, the present invention will be specifically described with reference to Examples, but the present invention is not limited thereto. First, measuring methods and evaluation methods are shown below.

(Method of determining peaks derived from microcrystals)

In the Second Hutch at BL03XU of SPring-8, the porous fiber was fixed to an auto sample changer, and irradiated with X-rays having a beam diameter of approximately 100 μm and a wavelength of 0.1 nm in the direction perpendicular to the fiber axis. A wide-angle X-ray scattering image obtained by the SOPHIAS detector was one-dimensionalized using graph processing software Igor Pro (registered trademark, manufactured by WaveMetrics, Inc.) in such a manner that the horizontal axis was the scattering vector q and the vertical axis was the X-ray scattering intensity.

The camera length is approximately 50 mm to 90 mm, and is arbitrarily determined within a range where scattering derived from the target microcrystals can be observed. After one-dimensionalization, background removal was appropriately performed in such a manner that only scattering derived from the porous fiber remained. Specifically, in a case where there was nothing on the optical path other than the porous fiber, the background was removed using air scattering, and in a case where the porous fiber was placed in a cell including water, a film, and the like for measurement-for purposes such as preventing drying-the background was removed using the scattering from an empty cell.

Additionally, in a case where the porous fiber contains a third component such as fine particles other than the organic polymer, background removal is also appropriately performed for scattering derived from the third component. Regarding a peak of a scattering spectrum after background removal, peak assignment was performed through literature or X-ray structural analysis, and in a case where even one was attributed as a peak derived from microcrystals, it was determined that microcrystals were present.

For example, it is known that the scattering vector of the amorphous-derived peak of PMMA is $q = 0.95$ Å$^{-1}$, and that in the case of PMMA stereocomplex microcrystals, the stereocomplex peaks are at $q = 0.29$, 0.84, and 1.14 Å$^{-1}$ (Macromolecules, 2021, 54, 2001-2010).

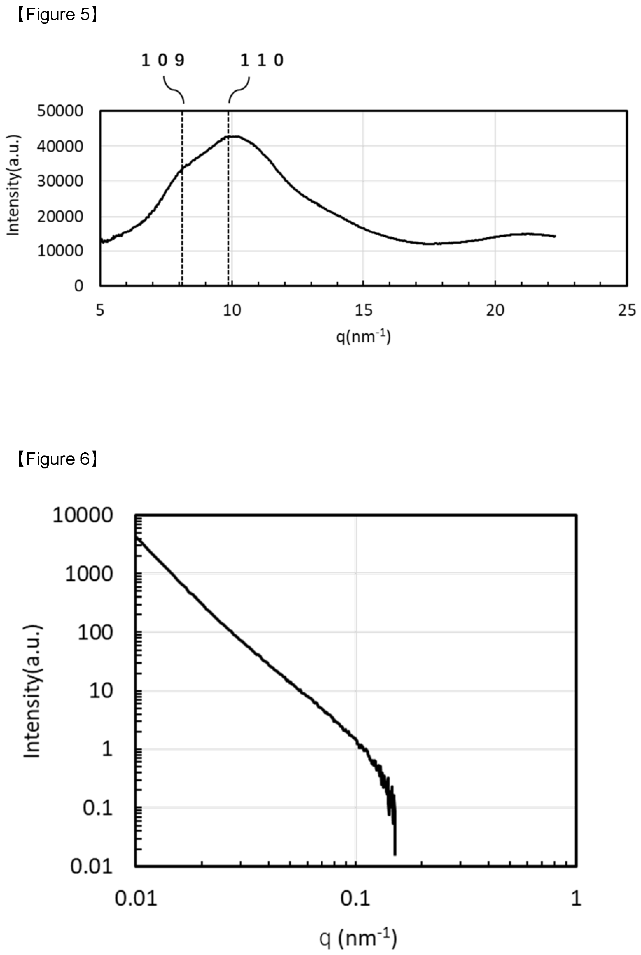

Accordingly, if a peak is present at at least one of the vicinities of $q = 0.29$, 0.84, and 1.14 Å$^{-1}$ (within an error range of $\pm 0.05$ Å$^{-1}$), it is determined that microcrystals are observed. Additionally, as shown in FIG. 5, also in a case where a peak derived from microcrystals overlaps with a peak 110 derived from an amorphous portion to form a shoulder peak 109 derived from microcrystals, it is determined that microcrystals have been observed. In this case, peak separation by fitting is performed, and if a peak remains (that is, is convex upward) near the peak position derived from microcrystals in the residual after fitting the peak derived from the amorphous portion, it can be determined that microcrystals are present.

For example, regarding PMMA stereocomplex microcrystals, if a peak remains (is convex upward) in at least one of the vicinities of $q = 0.29$, 0.84, and 1.14 Å$^{-1}$ in the residual after performing fitting with $q = 0.95$ Å$^{-1}$ as the peak position, it is determined that stereocomplex microcrystals are present.

Conversely, if a peak does not remain (is not convex upward) at the peak position derived from microcrystals in the residual after fitting the peak derived from the amorphous portion, it is determined that microcrystals are not present. For example, regarding PMMA stereocomplex microcrystals, if a peak did not remain (was not convex upward) at any position in the vicinities of $q = 0.29$, 0.84, and 1.14 Å$^{-1}$ in the residual after performing fitting with $q = 0.95$ Å$^{-1}$ as the peak position, it was determined that there were no microcrystals.

In a case where it is determined that a peak derived from microcrystals is present, the accurate peak position of the peak derived from microcrystals is determined by fitting. For example, in a case where a peak is present near 0.84 Å$^{-1}$, fitting is performed with $q = 0.84$ Å$^{-1}$ as the peak position to determine the peak position. In a case where a peak is also present near other peaks derived from microcrystals (for example, $q = 1.14$ Å$^{-1}$) or near $q = 0.95$ Å$^{-1}$ which is derived from the amorphous portion, forming a shoulder peak, the accurate peak position of the peak derived from microcrystals was determined by performing peak separation by fitting for the plurality of peaks present. The function used for fitting was a Gaussian function, and the fitting range was appropriately determined according to the observed peak width. Additionally, background correction during fitting was performed using a linear function. Note that the peak position was determined to be the relevant peak if the peak was within an error range of $q = \pm 0.05$ Å$^{-1}$.

(Average pore diameter near outer surface in cross section of fiber)

A sufficiently moistened porous fiber was placed in a container filled with water, frozen with liquid nitrogen, and a block in which the porous fiber was embedded was obtained. The block was sliced to a thickness of 200 nm at a temperature of -65°C using an ultramicrotome with a cryo-system to obtain a section in which the cross section in the radial

direction of the porous fiber was exposed.

The obtained section was freeze-dried in a vacuum dryer at 0.1 torr or lower to sublimate water, obtaining a dried sample. Then, a thin film of platinum-palladium (Pt-Pd) was formed on the sample surface by sputtering, and used as an observation sample.

The cross section of the fiber of the sample was observed with a scanning electron microscope (S-5500 manufactured by Hitachi High-Technologies Corporation). When an arbitrary straight line was drawn from the center point to the outer periphery in the cross section of the fiber, a region corresponding to the 10% portion on the outer periphery side was observed and imaged at a magnification at which pores was able to be sufficiently observed (10,000 times to 20,000 times). However, imaging was performed excluding the slight dense layer region existing on the outermost surface.

After imaging, image analysis software (ImageJ) was used to perform binarization processing in such a manner that the pore portion was black and the structure portion was white, and the total area of the pores was determined. In a case where binarization processing of the pore portion and the structure portion was difficult owing to the contrast difference of the electron microscope image, a transparent sheet was overlaid on the printed image, and the void portion was painted black using a black pen or the like. Then, an image obtained by copying the transparent sheet onto white paper was used to perform binarization processing in such a manner that the pore portion was black and the structure portion was white.

Note that a black portion having five or fewer continuous pixels, in which portion noise and pores were not able to be distinguished, was treated as a white portion, which is a structure. From the area of each pore, the equivalent circle diameter, which is the diameter of a circle having the same area, was calculated. The obtained equivalent circle diameters were arranged in descending order. The areas of the pores were added in order from the pore having the largest equivalent circle diameter. The equivalent circle diameter of the pore when the sum exceeded 1/2 of the total area of all the pores was defined as the cross-sectional pore diameter.

The cross-sectional pore diameter was calculated in the same manner at three arbitrary locations within the region near the outer surface, and the average value thereof was defined as the average pore diameter near the outer surface in the cross section of the fiber. Note that for the average pore diameter, a value rounded to the nearest integer was used.

(Average pore diameter at or near center in cross section of fiber)

In the above-described "Average pore diameter near the outer surface in a cross section of the fiber", the same operation was performed except that the region to be observed and imaged was at or near the center of the porous fiber, and the average pore diameter at or near the center in a cross section of the fiber was calculated.

(Average pore diameter of intermediate region in cross section of fiber)

In the above-mentioned "Average pore diameter near the outer surface in a cross section of the fiber", the same operation was performed except that the region to be observed and imaged was the intermediate region of the porous fiber, and the average pore diameter of the intermediate region in a cross section of the fiber was calculated.

(Average pore diameter in cross section of fiber)

An arbitrary straight line is drawn from the center point to the outer periphery in a cross section of the fiber, and the straight line is divided into 10 equal parts to divide the cross section of the fiber into 10 small regions. The average pore diameter in the cross section of the fiber is expressed by the following Formula (1), wherein $R_1$ to $R_{10}$ are the average pore diameters of the respective small regions in order starting from the region closest to the center point, and $S_1$ to $S_{10}$ are the areas of the respective small regions.

[Math 1]

$$\sum_{j=1}^{10}(R_j \times S_j) \Big/ \sum_{j=1}^{10} S_j \quad \text{... Formula (1)}$$

The average pore diameter $R_j$ of each small region is calculated by performing the same operation except that the region to be observed and imaged is set to each small region in the above-described "Average pore diameter near the outer surface in a cross section of the fiber".

(Porosity of outer surface of fiber)

A sufficiently moistened porous fiber was frozen with liquid nitrogen, and freeze-dried in a vacuum dryer at 0.1 torr or lower for 24 hours to sublimate water, thereby obtaining a dried sample. Then, a thin film of platinum-palladium (Pt-Pd) was formed on the sample surface by sputtering, and used as a surface observation sample.

The fiber surface of the surface observation sample was observed with a scanning electron microscope (S-5500 manufactured by Hitachi High-Technologies Corporation), and an arbitrary position on the fiber surface was imaged. The magnification was set to a magnification at which pores were able to be sufficiently recognized (5,000 times to 10,000 times). After imaging, the pores contained in each image were measured, and the total area of all the pores was determined.

For the total area of all the pores, the electron microscope image was subjected to binarization processing using image analysis software "ImageJ" in such a manner that the pore portion was black and the non-pore portion was white. The obtained image was analyzed to calculate the total area of all the pores. In a case where binarization processing of the pore portion and the non-pore portion was difficult owing to the contrast difference of the electron microscope image, a transparent sheet was overlaid on the printed image, and the void portion was painted black using a black pen or the like. Then, an image obtained by copying the transparent sheet onto white paper was used to perform binarization processing in such a manner that the pore portion was black and the non-pore portion was white. Then, analysis was performed.

From the total area of all the obtained pores and the area of the entire image, the porosity of the outer surface of the fiber was calculated by the following Formula (2). This measurement was performed at 9 locations spaced by an interval of 1 cm or more in the longitudinal direction of the fiber. The values were averaged, and the resultant value was rounded to the first decimal place.

Porosity of the outer surface of the fiber (%) = Total area of all pores ($\mu m^2$) / Area of the entire image ($\mu m^2$) $\times$ 100 Formula (2)

(Average pore diameter in outer surface of fiber)

Using the binarized image obtained in the above-mentioned "Porosity of the outer surface of the fiber", the equivalent circle diameter converted from the area of each pore was calculated. The equivalent circle diameters were arranged in descending order. The areas of the pores were added in order from the pore having the largest equivalent circle diameter. The equivalent circle diameter of the pore when the sum exceeded 1/2 of the total area of all the pores was defined as the pore diameter of the surface.

Using nine or more images spaced by an interval of 1 cm or more in the longitudinal direction of the fiber, the equivalent circle diameters were calculated in the same manner, and the average value thereof was defined as the average pore diameter in the outer surface of the fiber. Note that for the average pore diameter in the outer surface of the fiber, a value rounded to the nearest integer was used.

(Aspect ratio of major axis portion and minor axis portion of pores in outer surface of fiber)

Using the binarized image obtained in the above-described "Porosity of the outer surface of the fiber", 15 or more pores per image were fitted with ellipses, and the ratio of major axis / minor axis was calculated. The same measurement was performed on nine or more images spaced by an interval of 1 cm or more in the longitudinal direction of the fiber. The values were averaged, and the aspect ratio of the major axis portion and the minor axis portion of the pores in the outer surface of the fiber was calculated.

(Viscosity of spinning dope)

The viscosity is measured by a falling ball method in a constant temperature bath set to a high temperature according to JIS Z 8803 (2011 edition). Specifically, the viscosity is obtained by filling a viscometer tube having an inner diameter of 40 mm with the spinning dope, dropping a steel ball (made of SUS316) having a diameter of 2 mm into the dope, and measuring the time required for the ball to fall 200 mm. The temperature at the time of measurement is 110°C.

(Gel point of spinning dope)

The gel point of the spinning dope was determined in the above-mentioned "Viscosity of the spinning dope" as the temperature when the viscosity suddenly increased while the measurement temperature of the viscosity was lowered from 110°C at 5°C intervals during the measurement of the viscosity. Specifically, the temperature when the viscosity reached 10 times or more the viscosity at 110°C was taken as the gel point.

(Ultra-small angle X-ray scattering of spinning dope)

A through-hole was made in a 0.5 mm thick silicone rubber sheet, and the spinning dope was enclosed in a measurement cell covered with Kapton film on both sides. In the Second Hutch at BL03XU of SPring-8, the measurement cell containing the spinning dope was fixed to a cooling/heating stage (Linkam Scientific Instruments Limited).
After heating the measurement cell to 110°C, while lowering the temperature at 5°C/min, X-rays with a beam diameter of approximately 100 μm and a wavelength of 0.2 nm were irradiated 20 times at 1-minute intervals. The camera length was 7.82 m, the integration time was 10 s, and the attenuator was Al 25 nm.
The ultra-small angle X-ray scattering image obtained by the PILATUS detector was one-dimensionalized using graph processing software Igor Pro (registered trademark, manufactured by WaveMetrics, Inc.) to create a log-log graph in such a manner that the horizontal axis was the scattering vector q, and the vertical axis was the scattering intensity.
For a reference in which only the solvent of the spinning dope was enclosed in the measurement cell, ultra-small angle X-ray scattering was similarly measured under the same conditions, and background processing was performed. Regarding the scattering spectrum after background processing, in a region where q = 1 $nm^{-1}$ or less, as shown in FIG. 6, when a region exists in which the scattering intensity becomes stronger as q becomes smaller, it was determined that X-ray scattering was observed.

(Spinnability)

The spinnability was judged as follows.

○: When the porous fiber was taken up at a spinning speed of 5 m/min, 150 m was taken up without thread breakage.
△: When the porous fiber was taken up at a spinning speed of 5 m/min, 150 m was subjected to thread breakage one to three times.
×: When the porous fiber was taken up at a spinning speed of 5 m/min, 150 m was subjected to thread breakage four or more times.

(Dispersion component of surface free energy)

Freeze-dried porous fibers were packed in a glass column. Using a surface free energy measuring device iGC-SEA manufactured by Nippon Science Core Co., the dispersion component of surface free energy was measured and calculated with reference to the method described in "Inverse gas chromatography applications: A review" Adv. Colloid Interface Sci., 212, 21-44 (2014). Specifically, a straight-chain alkane having 6 to 9 carbon atoms was used as a test solvent, and the retention volume was calculated from the obtained chromatogram. The dispersion component of the surface free energy was calculated from the retention volumes of various alkanes.

(Adsorption performance of porous fiber)

A porous fiber was fixed and cut while applying a tension of 0.01 to 0.10 g/$mm^2$ to obtain a section in which a cross section in the radial direction of the porous fiber was exposed. The cut surface was enlarged with an optical microscope and photographed. Using image analysis software, the outer peripheral part of the fiber cross section was traced to calculate the perimeter length P (cm).
A sample for an adsorption test was prepared by finely cutting 200 porous fibers cut to a length of 5.5 cm in the longitudinal direction of the fiber. Note that the total surface area S ($cm^2$) of the sample for an adsorption test was calculated by the following Formula (3-1).

$$S\ (cm^2) = P\ (cm) \times 5.5\ (cm) \times 200 \ \ldots \text{Formula (3-1)}$$

The sample for an adsorption test having the above total surface area was placed in a 5 mL container, and 2.6 mL of commercially available human plasma was added thereto. The resultant mixture was shaken and stirred at 37°C for 4 hours using a seesaw shaker (Wave-SI manufactured by TAITEC Corporation) with the scale set to 38 and the angle set to maximum (1 reciprocation in 1.7 seconds). Human plasma before contact with the porous fiber and human plasma after completion of stirring, 1 mL each, were sampled.
LDL cholesterol was measured by a direct method, the LDL cholesterol concentration C1 (mg/ml) in human plasma before contact with the porous fiber and the LDL cholesterol concentration C2 (mg/ml) after completion of stirring were obtained, and the adsorption amount per fiber surface area was calculated by the following Formula (3-2).

Adsorption amount of LDL cholesterol per fiber surface area ($mg/cm^2$) = (C1 - C2) (mg/ml) × 2.6 (mL) / S ($cm^2$)    Formula (3-2)

Note that for each adsorption amount, a value obtained by averaging the values measured three times and rounding the resultant average off to the second decimal place was used.

[Example 1]

A spinning dope was prepared by mixing 200 parts by weight of syn-PMMA having a weight average molecular weight of 1,400,000, 40 parts by weight of iso-PMMA having a weight average molecular weight of 500,000, 1,560 parts by weight of DMSO, and 170 parts by weight of formamide in such a manner that the PMMA concentration was 12% by weight. The obtained spinning dope had a gel point of 90°C and a viscosity at 110°C of 175.5 Pa·s.

The spinning dope was discharged into the air using a circular spinneret. The spinneret temperature was set to 95°C. The dry length, namely, the length of the air-traveling section, was 50 cm. The fiber was cooled at a cold air speed of 30 $m^3$/h during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 1.

[Example 2]

A spinning dope was prepared by mixing 200 parts by weight of syn-PMMA having a weight average molecular weight of 1,400,000, 40 parts by weight of iso-PMMA having a weight average molecular weight of 500,000, 1,627 parts by weight of DMSO, and 88 parts by weight of 1,4-butanediol in such a manner that the PMMA concentration was 12% by weight. The obtained spinning dope had a gel point of 75°C and a viscosity at 110°C of 201.0 Pa·s.

The spinning dope was discharged into the air using a circular spinneret. The spinneret temperature was set to 95°C. The length of the air-traveling section was 50 cm. The fiber was cooled at a cold air speed of 30 $m^3$/h during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 2.

[Example 3]

A spinning dope was prepared by mixing 200 parts by weight of syn-PMMA having a weight average molecular weight of 1,400,000, 40 parts by weight of iso-PMMA having a weight average molecular weight of 500,000, 1,627 parts by weight of DMSO, and 88 parts by weight of propylene glycol in such a manner that the PMMA concentration was 12% by weight. The obtained spinning dope had a gel point of 75°C and a viscosity at 110°C of 242.0 Pa·s.

The spinning dope was discharged into the air using a circular spinneret. The spinneret temperature was set to 95°C. The length of the air-traveling section was 50 cm. The fiber was cooled at a cold air speed of 30 $m^3$/h during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 3.

[Example 4]

A spinning dope was prepared by mixing 200 parts by weight of syn-PMMA having a weight average molecular weight of 1,400,000, 40 parts by weight of iso-PMMA having a weight average molecular weight of 500,000, 1,627 parts by weight of DMSO, and 88 parts by weight of glycerin in such a manner that the PMMA concentration was 12% by weight. The obtained spinning dope had a gel point of 75°C and a viscosity at 110°C of 230.0 Pa·s.

The spinning dope was discharged into the air using a circular spinneret. The spinneret temperature was set to 95°C. The length of the air-traveling section was 50 cm. The fiber was cooled at a cold air speed of 33 $m^3$/h during air-traveling. Then, the fiber was introduced into a coagulation bath containing water at 70°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 4.

[Example 5]

A porous fiber 5 was obtained by performing the same operations as in Example 4, except that the cold air speed was 36 $m^3$/h and the coagulation bath temperature was 60°C.

[Example 6]

A spinning dope was prepared by mixing 178 parts by weight of syn-PMMA having a weight average molecular weight of 1,400,000, 36 parts by weight of iso-PMMA having a weight average molecular weight of 500,000, 2,362 parts by weight of DMSO, and 184 parts by weight of glycerin in such a manner that the PMMA concentration was 7.8% by weight. The obtained spinning dope had a gel point of 60°C and a viscosity at 110°C of 25.9 Pa·s.

The spinning dope was discharged into the air using a syringe pump. The syringe temperature was set to 90°C. The length of the air-traveling section was 70 cm. The fiber was cooled at a cold air speed of 30 $m^3$/h during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 3 m/min to obtain a porous fiber 6.

[Example 7]

A porous fiber 7 was obtained by performing the same operations as in Example 5, except that the winding speed was 10 $m^3$/h.

[Example 8]

A porous fiber 8 was obtained by performing the same operations as in Example 5, except that the winding speed was 3 $m^3$/h.

[Example 9]

A spinning dope a for forming a region A was prepared by mixing 40.9 parts by mass of syn-PMMA having a weight average molecular weight of 1,400,000, 6.8 parts by mass of iso-PMMA having a weight average molecular weight of 500,000, and 428 parts by mass of dimethyl sulfoxide (hereinafter referred to as "DMSO") in such a manner that the PMMA concentration was 10% by mass, and stirring the resultant mixture at 110°C for 8 hours. The obtained spinning dope had a viscosity at 110°C of 190 poise.

Additionally, in the same manner, a spinning dope b for forming a region B was prepared by mixing 31.7 parts by mass of syn-PMMA having a weight average molecular weight of 400,000, 31.7 parts by mass of syn-PMMA having a weight average molecular weight of 1,400,000, 16.7 parts by mass of iso-PMMA having a weight average molecular weight of 500,000, 20 parts by mass of a PMMA copolymer having a molecular weight of 300,000 and containing 1.5 mol% of sodium para-styrenesulfonate having a sulfo group showing a negative charge, and 376 parts by mass of DMSO in such a manner that the PMMA concentration was 15% by mass, and stirring the resultant mixture at 110°C for 8 hours. The obtained spinning dope had a viscosity at 110°C of 102 poise.

The dopes were discharged simultaneously into the air at a rate of 1.0 cc/min for both using a concentric core-sheath spinneret in such a manner that the spinning dope a formed a sheath and the spinning dope b formed a core. The spinneret temperature was set to 99°C. The length of the air-traveling section was 55 cm, and the air-traveling time was 0.72 seconds. The fiber was cooled at a cold air speed of 7.5 m/second during air-traveling. Then, the fiber was introduced into a coagulation bath containing a 20% by mass DMSO aqueous solution at 60°C. The obtained composite porous fiber was washed with water in a water bath at 40°C. Subsequently, the porous fiber was introduced into a bath containing a 70% by mass glycerin aqueous solution for moisturizing. The porous fiber was passed through a heat treatment bath at 85°C to remove excess glycerin, and then wound up at 20 m/min to obtain a porous fiber 9.

[Comparative Example 1]

A spinning dope was prepared by mixing 171 parts by weight of syn-PMMA having a weight average molecular weight of 1,400,000, 34 parts by weight of iso-PMMA having a weight average molecular weight of 500,000, 2,159 parts by weight of DMSO, and 263 parts by weight of glycerin in such a manner that the PMMA concentration was 7.8% by weight. The obtained spinning dope had a gel point of 65°C and a viscosity at 110°C of 107.7 Pa·s.

The spinning dope was discharged into the air using a syringe pump. The syringe temperature was set to 90°C.

The length of the air-traveling section was 70 cm. The fiber was cooled at a cold air speed of 30 $m^3/h$ during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 3 m/min to obtain a porous fiber 9. Fiber breakage occurred frequently, and the spinnability was poor.

[Comparative Example 2]

A spinning dope was prepared by mixing 290 parts by weight of syn-PMMA having a weight average molecular weight of 1,400,000, 58 parts by weight of iso-PMMA having a weight average molecular weight of 500,000, and 2,554 parts by weight of DMSO in such a manner that the PMMA concentration was 12% by weight. The obtained spinning dope had a gel point of 65°C and a viscosity at 110°C of 154.6 Pa·s.

The spinning dope was discharged into the air using a circular spinneret. The spinneret temperature was set to 95°C. The length of the air-traveling section was 50 cm. The fiber was cooled at a cold air speed of 30 $m^3/h$ during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 10.

[Comparative Example 3]

A spinning dope was prepared by mixing 31.7 parts by weight of syn-PMMA having a weight average molecular weight of 400,000, 31.7 parts by weight of syn-PMMA having a weight average molecular weight of 1,400,000, 16.7 parts by weight of iso-PMMA having a weight average molecular weight of 500,000, 20 parts by weight of a PMMA copolymer having a molecular weight of 300,000 and containing 1.5 mol% of sodium para-styrenesulfonate having a sulfo group showing a negative charge, and 376 parts by weight of DMSO in such a manner that the PMMA concentration was 21% by weight. The obtained spinning dope had a gel point of 70°C and a viscosity at 110°C of 423.0 Pa·s.

The spinning dope was discharged into the air using a circular spinneret. The spinneret temperature was set to 95°C. The length of the air-traveling section was 50 cm. The fiber was cooled at a cold air speed of 30 $m^3/h$ during air-traveling. Then, the fiber was introduced into a coagulation bath containing water at 40°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 20 m/min to obtain a porous fiber 11.

[Comparative Example 4]

A porous fiber 12 was obtained by performing the same operations as in Example 5, except that the cold air speed was 42 $m^3/h$.

[Comparative Example 5]

A spinning dope was prepared by mixing 168 parts by weight of syn-PMMA having a weight average molecular weight of 1,400,000, 34 parts by weight of iso-PMMA having a weight average molecular weight of 500,000, 2,167 parts by weight of DMSO, and 329 parts by weight of formamide in such a manner that the PMMA concentration was 7.5% by weight. The obtained spinning dope had so high viscosity that the viscosity at 110°C of the dope was unmeasurable.

The spinning dope was discharged into the air using a syringe pump. The syringe temperature was set to 90°C. The length of the air-traveling section was 70 cm. The fiber was cooled at a cold air speed of 30 $m^3/h$ during air-traveling. Then, the fiber was introduced into a coagulation bath containing water at 60°C. The obtained porous fiber was washed with water in a water bath at 40°C. Subsequently, the porous fiber was introduced into a bath containing a 70% by weight glycerin aqueous solution for moisturizing. The porous fiber was passed through a heat treatment bath at 85°C to remove excess glycerin, and then wound up at 3 m/min to obtain a porous fiber 13. Fiber breakage occurred frequently, and the spinnability was poor.

[Comparative Example 6]

A porous fiber 14 was obtained by performing the same operations as in Example 5, except that the length of the air-traveling section was 0.3 cm and the cold air volume was 0 $m^3/h$.

Spinning conditions of the obtained Examples 1 to 9 are shown in Table 1, and spinning conditions of Comparative Examples 1 to 6 are shown in Table 2. Additionally, evaluation results of Examples 1 to 9 are shown in Table 3, and evaluation results of Comparative Examples 1 to 6 are shown in Table 4.

[Table 1]

| | | | Example 1 | Example 2 | Example 3 | Example 4 | Example 5 | Example 6 | Example 7 | Example 8 | Example 9 | |
|---|---|---|---|---|---|---|---|---|---|---|---|---|
| Spinning conditions | Spinning dope | Polymer concentration (%) | 12 | 12 | 12 | 12 | 12 | 7.8 | 12 | 12 | 10 | 15 |
| | | Good solvent | DMSO | DMSO | DMSO | DMSO | DMSO | DMSO | DMSO | DMSO | DMSO | DMSO |
| | | Poor solvent | Formamide | 1,4-butanediol | Propylene glycol | Glycerin | Glycerin | Glycerin | Glycerin | Glycerin | - | - |
| | | Poor solvent / (Good solvent + Poor solvent) weight ratio (%) | 10 | 5 | 5 | 5 | 5 | 7.2 | 5 | 5 | - | - |
| | | Viscosity (Pa·s) | 175.5 | 201.0 | 242.0 | 230.0 | 230.0 | 25.9 | 230.0 | 230.0 | 190 | 102 |
| | | Gel point (°C) | 90 | 75 | 75 | 75 | 75 | 60 | 75 | 75 | 60 | 60 |
| | | Spinnability | ○ | ○ | ○ | ○ | ○ | ○ | ○ | ○ | ○ | |
| | | Ultra-small angle X-ray scattering at gel point or higher | Yes | Yes | Yes | Yes | Yes | Yes | Yes | Yes | None | |
| | Cold air volume ($m^3/h$) | | 30 | 30 | 30 | 33 | 36 | 30 | 36 | 36 | 30 | |
| | Coagulation bath temperature (°C) | | 60 | 60 | 60 | 70 | 60 | 60 | 60 | 60 | 60 | |
| | Dry length (cm) | | 50 | 50 | 50 | 50 | 50 | 70 | 50 | 50 | 50 | |
| | Spinning speed (m/min) | | 20 | 20 | 20 | 20 | 20 | 3 | 10 | 3 | 20 | |

[Table 2]

| | | | Comparative Example 1 | Comparative Example 2 | Comparative Example 3 | Comparative Example 4 | Comparative Example 5 | Comparative Example 6 |
|---|---|---|---|---|---|---|---|---|
| Spinning conditions | Spinning dope | Polymer concentration (%) | 7.8 | 12 | 21 | 12 | 7.5 | 12 |
| | | Good solvent | DMSO | DMSO | DMSO | DMSO | DMSO | DMSO |
| | | Poor solvent | Glycerin | - | - | Glycerin | Formamide | Glycerin |
| | | Poor solvent / (Good solvent + Poor solvent) weight ratio (%) | 11 | - | - | 5 | 15 | 5 |
| | | Viscosity (Pa·s) | 107.7 | 154.6 | 423.0 | 230.0 | High viscosity, Unmeasurable | 230.0 |
| | | Gel point (°C) | 65 | 65 | 70 | 75 | - | 75 |
| | | Spinnability | × | ○ | ○ | ○ | × | ○ |
| | | Ultra-small angle X-ray scattering at gel point or higher | Yes | None | None | Yes | Yes | Yes |
| | Cold air volume ($m^3/h$) | | 30 | 30 | 30 | 42 | 30 | 0 |
| | Coagulation bath temperature (°C) | | 60 | 60 | 40 | 60 | 60 | 60 |
| | Dry length (cm) | | 70 | 50 | 50 | 50 | 70 | 0.3 |
| | Spinning speed (m/min) | | 3 | 20 | 20 | 20 | 3 | 20 |

[Table 3]

| | | | | Example 1 | Example 2 | Example 3 | Example 4 | Example 5 | Example 6 | Example 7 | Example 8 | Example 9 |
|---|---|---|---|---|---|---|---|---|---|---|---|---|
| Yarn properties | Porous structure | Cross section of fiber | Average pore diameter (nm) | 202 | 105 | 110 | 195 | 225 | 723 | 175 | 372 | 101 |
| | | | Average pore diameter at or near center (Ri) (nm) | 167 | 81 | 97 | 184 | 157 | 522 | 165 | 321 | 65 |
| | | | Average pore diameter near outer surface (Ro) (nm) | 350 | 152 | 126 | 222 | 354 | 843 | 371 | 479 | 125 |
| | | | Ro/Ri | 2.1 | 1.9 | 1.3 | 1.2 | 2.3 | 1.6 | 1.6 | 1.5 | 1.9 |
| | | | Average pore diameter in intermediate region (Rm) (nm) | 179 | 85 | 102 | 190 | 168 | 561 | 171 | 350 | 67 |
| | | | Rm/Ri | 1.07 | 1.05 | 1.05 | 1.03 | 1.07 | 1.07 | 1.04 | 1.09 | 1.03 |
| | | Outer surface of fiber | Average pore diameter (nm) | 385 | 637 | 328 | 479 | 661 | 839 | 423 | 475 | 310 |
| | | | Porosity (%) | 10.2 | 5.5 | 5.1 | 7.0 | 12.0 | 14.7 | 7.9 | 5.4 | 13.0 |
| | | | Major axis/minor axis aspect ratio of pore | 4.2 | 5.2 | 2.5 | 3.2 | 4.5 | 1.5 | 1.7 | 1.3 | 2.9 |
| | Structural analysis | Peak position derived from microcrystal ($Å^{-1}$) | | 0.83 | 0.81 | 0.81 | 0.81 | 0.82 | 0.83 | 0.82 | 0.83 | No microcrystal |
| | Dispersion component of surface free energy ($mJ/m^2$) | | | 50 | 50 | 50 | 50 | 50 | 50 | 50 | 50 | 50 |
| Adsorption performance | Adsorption amount of LDL per fiber surface area ($\mu g/cm^2$) | | | 3.2 | 1.6 | 1.7 | 1.9 | 3.1 | 10.5 | 1.8 | 1.6 | 1.8 |

[Table 4]

| | | | | Comparative Example 1 | Comparative Example 2 | Comparative Example 3 | Comparative Example 4 | Comparative Example 5 | Comparative Example 6 |
|---|---|---|---|---|---|---|---|---|---|
| Yarn properties | Porous structure | Cross section of fiber | Average pore diameter (nm) | 1124 | 89 | 19 | 191 | 1055 | 287 |
| | | | Average pore diameter at or near center (Ri) (nm) | 781 | 106 | 18 | 144 | 675 | 297 |
| | | | Average pore diameter at or near outer surface (Ro) (nm) | 1347 | 67 | 20 | 271 | 1133 | 283 |
| | | | Ro/Ri | 1.72 | 0.63 | 1.11 | 1.9 | 1.68 | 0.95 |
| | | | Average pore diameter in intermediate region (Rm) (nm) | 863 | 95 | 18 | 149 | 804 | 284 |
| | | | Rm/Ri | 1.10 | 0.90 | 1.00 | 1.03 | 1.19 | 0.96 |
| | | Outer surface of fiber | Average pore diameter (nm) | 1055 | 489 | 55 | 370 | 1229 | No pore |
| | | | Porosity (%) | 18.1 | 5.3 | 3.0 | 3.8 | 12.9 | 0 |
| | | | Major axis/minor axis aspect ratio of pore | 1.5 | 4.4 | 1.1 | 4.1 | 1.8 | No pore |
| | Structural analysis | Peak position derived from microcrystal ($Å^{-1}$) | | 0.83 | No microcrystal | No microcrystal | 0.82 | 0.83 | No microcrystal |
| | Dispersion component of surface free energy ($mJ/m^2$) | | | 50 | 50 | 50 | 50 | 50 | 50 |
| Adsorption performance | Adsorption amount of LDL per fiber surface area ($\mu g/cm^2$) | | | - | 0.9 | 0.5 | 1.0 | - | 1.0 |

Industrial Applicability

Examples of applications of the porous fiber of the present invention include: filters for various fluids regardless of whether the fluids are in a gaseous or liquid phase; adsorbents; heat insulators; sound absorbing materials; impact buffering materials; base materials for cell culture; and carriers for regenerative medicine. In particular, in medical applications, the adsorption column is suitably used for the removal of pathogenic proteins and the like from blood, plasma, and body fluids and for the removal of impurities from biopharmaceutical raw material solutions for use in the production of biopharmaceuticals.

Reference Signs List

100 Virtual region 1
101 Virtual region 2
102 Virtual region boundary
103 Perpendicular line to a tangent line at an arbitrary point of the virtual region boundary
104 Boundary of region 1
105 Boundary of region 2
106 Region boundary
107 Region 1
108 Region 2
109 Shoulder peak derived from microcrystals
110 Peak derived from an amorphous portion

**Claims**

**1.** A porous fiber comprising an organic polymer,

wherein the fiber has an average pore diameter in a cross section of 100 to 1000 nm,
wherein the fiber has an average pore diameter in an outer surface of 100 to 10,000 nm,
wherein Rm/Ri is 0.85 to 1.15, wherein Rm is average pore diameter in an intermediate region of the cross section of the fiber, and Ri is average pore diameter at or near the center of the cross section of the fiber, and
wherein the outer surface of the fiber has a porosity of 5 to 50%.

**2.** The porous fiber according to claim 1, wherein the porosity of the outer surface of the fiber is 5 to 40%.

**3.** The porous fiber according to claim 1, wherein aspect ratio of major axis portion to minor axis portion of a pore in the outer surface of the fiber is 1.5 to 10.

**4.** The porous fiber according to claim 1, wherein the fiber has a dispersion component of a surface free energy of 5 to 100 $mJ/m^2$.

**5.** The porous fiber according to claim 1, wherein the organic polymer is an amorphous polymer.

**6.** The porous fiber according to claim 5, wherein the amorphous polymer is an acrylic polymer.

**7.** The porous fiber according to claim 5, wherein the amorphous polymer is polymethyl methacrylate.

**8.** The porous fiber according to claim 1,

wherein the cross section of the fiber has a region A and a region B separated by a region boundary,
wherein the region A is a uniform structure having an average pore diameter Ap of 100 to 1000 nm, and
wherein an average pore diameter Bp of the region B is smaller than the average pore diameter Ap, or the region B is non-porous.

**9.** The porous fiber according to claim 1, wherein the porous fiber is a solid fiber.

**10.** A fiber bundle comprising the porous fiber according to claim 1.

**11.** An adsorption column comprising the fiber bundle according to claim 10.

**12.** The adsorption column according to claim 11, for use in the blood purification.

**13.** A method of producing a pharmaceutical, comprising a purification step using the adsorption column according to claim 10.

【Figure 1】

102
100
101
S5500 3.0kV x400 SE
100um

【Figure 2】

100
101
102
103
S5500 3.0kV x10.0k SE
5.00um

【Figure 3】

104
105
107
106
108
Pore diameter
Position

【Figure 4】

Pore diameter
107
106
108
Pore diameter
107
106
108
Pore diameter
107
106
108

(Fig. 4-A)

(Fig. 4-B)

(Fig. 4-C)

【Figure 5】

109
110
50000
40000
30000
20000
10000
0
Intensity(a.u.)
5
10
15
20
25
q(nm-1)

【Figure 6】

10000
1000
100
10
1
0.1
0.01
Intensity(a.u.)
0.01
0.1
1
q (nm-1)

## INTERNATIONAL SEARCH REPORT

International application No. **PCT/JP2025/005803**

### A. CLASSIFICATION OF SUBJECT MATTER

***D01F 6/00***(2006.01)i; ***A61M 1/36***(2006.01)i; ***B01D 15/00***(2006.01)i; ***B01J 20/26***(2006.01)i; ***B01J 20/28***(2006.01)i; ***D01F 6/16***(2006.01)i; ***D01F 6/52***(2006.01)i

FI: D01F6/00 Z; A61M1/36 165; B01D15/00 K; B01J20/26 H; B01J20/28 A; D01F6/16; D01F6/52

According to International Patent Classification (IPC) or to both national classification and IPC

### B. FIELDS SEARCHED

Minimum documentation searched (classification system followed by classification symbols)

A61M1/36; B01D15/00-B01D15/42; B01J20/00-B01J20/34; D01F6/00-D01F8/18

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2012-92318 A (TORAY INDUSTRIES, INC.) 17 May 2012 (2012-05-17) claims 1, 4, example 2, table 1, paragraphs [0068]-[0070] | 1-2, 4-7, 9 |
| Y | | 3, 10-13 |
| A | | 8 |
| Y | JP 2-174921 A (TERUMO KABUSHIKI KAISHA) 06 July 1990 (1990-07-06) p. 9, lower right column, line 19 to p. 10, upper left column, line 4 | 3 |
| A | | 8 |
| Y | WO 2021/070857 A1 (TORAY INDUSTRIES, INC.) 15 April 2021 (2021-04-15) paragraphs [0001], [0050], [0061], [0077] | 10-13 |
| A | | 8 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 April 2025** | **22 April 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2025/005803**

| Patent document cited in search report | Publication date (day/month/year) | Patent family member(s) | Publication date (day/month/year) |
|---|---|---|---|
| JP 2012-92318 A | 17 May 2012 | JP 4962643 B2 | |
| JP 2-174921 A | 06 July 1990 | (Family: none) | |
| WO 2021/070857 A1 | 15 April 2021 | US 2022/0379000 A1 paragraphs [0001], [0068], [0079], [0096] | |
| | | CA 3156670 A1 | |
| | | CN 114531853 A | |
| | | EP 4026573 A1 | |
| | | KR 10-2022-0080084 A | |
| | | TW 202123987 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S5876104 A
- JP 2014207989 A
- WO 2017188119 A
- JP H06296860 A
- JP H07258915 A
- JP 2004098027 A


### Non-patent literature cited in the description


- *Macromolecules*, 2021, vol. 54, 2001-2010
- Inverse gas chromatography applications: A review. *Adv. Colloid Interface Sci.*, 2014, vol. 212, 21-44